Europäisches Patentamt

European Patent Office

Office européen des brevets

(11) Publication number: 0 163 489 A1

# (12) EUROPEAN PATENT APPLICATION

(21) Application number: 85303623.4

(22) Date of filing: 22.05.85

(51) Int. Cl.⁴: A 61 K 31/57
A 61 K 7/06

(30) Priority: 22.05.84 GB 8413069

(43) Date of publication of application:
04.12.85 Bulletin 85/49

(84) Designated Contracting States:
AT BE CH DE FR GB IT LI LU NL SE

(71) Applicant: Mortimer, Christopher Harry, Dr.
8 Park Square West Regents Park
London NW1 4LJ(GB)

(72) Inventor: Mortimer, Christopher Harry, Dr.
8 Park Square West Regents Park
London NW1 4LJ(GB)

(74) Representative: Baverstock, Michael George
Douglas et al,
BOULT, WADE & TENNANT 27 Furnival Street
London, EC4A 1PQ(GB)

(54) Treatment of female hair loss.

(57) A pharmaceutical formulation for use in treating female scalp hair loss or in hormonal replacement therapy which comprises medroxyprogesterone acetate or its pharmacologically-active equivalent and at least one oestrogen, wherein when prepared in unit dosage form the formulation is adapted to administer at least 1mg and not more than 500mg of metroxyprogesterone acetate or its pharmacologically-active equivalent per month, with the proviso that the said equivalent is not cyproterone acetate.

EP 0 163 489 A1

Croydon Printing Company Ltd.

## TREATMENT OF FEMALE HAIR LOSS

The present invention relates to the treatment of scalp hair loss and balding in women. This is a distressing condition which can affect women at the pre-, peri- post-menopause who have not previously experienced significant hair loss. Because thinning hair is not a socially acceptable condition in women, as it may be in men after a certain age, the condition can cause not merely distress but severe psyschological symtoms in women involving feelings of loss of femininity and self confidence, depression and inability to concentrate of anything except the prospect of impending baldness with mounting embarrassment.

Associated with the menopause, a number of so-called "menopausal symptoms" may be experienced by a patient. It has been found that the present treatment, designed for arresting hair loss, also alleviates these menopausal symptoms, and hence is also suitable for use in long-term hormonal replacement therapy.

The present invention provides a pharmaceutical formulation for use in treating female scalp hair loss or in hormonal replacement therapy which comprises medroxyprogesterone acetate or its pharmacologically-active equivalent and at least one oestrogen, wherein when prepared in unit dosage form the formulation is adapted to administer at least 1mg and not more than 500mg of medroxyprogesterone acetate or its pharmacologically-active equivalent per month, with the proviso that the said equivalent is not cyproterone acetate.

Medroxyprogesterone acetate is a synthetic progesterone-like preparation (17 alpha hydroxy - 6 alpha - methyl - delta 4 - pregnen-3, 20 dione - 17 alpha acetate) conveniently referred to as MPA. It has been used previously (as Provera) for the management of functional uterine bleeding, certain hormone dependent neoplasms (malignant diseases) such as endometrial carcinoma, hypernephroma, cancer of prostate.

It has now been found unexpectedly that scalp hair loss proceeding to baldness in women at the pre-, peri-, post-menopause can be arrested with the growth of new hair using a combination of at least one oestrogen with medroxyprogesterone acetate wherein, when prepared in unit dosage form, the formulation is adapted to administer at least 1mg and not more than 500mg of MPA per month. The invention further provides, in combination, a pharmaceutical formulation of the invention, a container therefor, and instructions for the use of the pharmaceutical formulation in the treatment of female scalp hair loss or in hormonal replacement therapy.

The formulations of the present invention may be designed for oral or parenteral administration. Although the formulations could be for administration by injection, they will most conveniently be for oral administration e.g. as tablets, capsules or solutions or suspensions.

They may however be formulated as creams or lotions for topical administration or as injectable solutions or suspensions for subcutaneous injection e.g. in the scalp or for other forms of injection. They may generally be formulated for any of the known routes of pharmaceutical administration.

Preferably, a formulation for oral administration will contain from 1 to 50mg, preferably 5 to 10mg, of MPA per unit dosage form e.g. per tablet.

The identity of the oestrogen used is not critical. Ethinyloestradiol is preferred. The oestrogen is present to provide a contraceptive effect, and in addition to provide the beneficial effect of raising plasma sex hormone binding globulin (SHBG) levels thereby diminishing free (biolofically-active) testosterone and other androgen levels. A suitable dose rate for ethinyloestradiol would be 10-60µg per day orally, preferably about 40µg.

The treatment of female scalp hair loss will preferably involve the administration of medroxyprogesterone acetate and the oestrogen in a cyclic manner. In the first part of the cycle, preferably the oestrogen alone will be administered, while in the second part of the cycle both oestrogen and MPA will be administered. This is followed preferably by no treatment to allow for withdrawal bleeding.

Preferably, the cycle length is 28 days in accordance with normal contraceptive practice.

A possible oral dosage regimen would therefore be:
Ethinyloestradiol 10-6-µg daily for 28 days of each cycle;
Medroxyprogesterone acetate 1-50mg daily for up to 27 days of the cycle;
or more preferably;
Ethinyloestradiol at 30-40µg daily for the first 21 days of each cycle and;
MPA at 5-10mg daily from days 16 to 21 of the cycle.

The dosage for oestrogens other than ethynyloestradiol will be well known to practioners.

Whilst the dosages of oestrogen and MPA for administration on the same day may conveniently be formulated together as a formulation according to the invention, they can equally well be taken separately.

In either case it will be advantageous if the two different types of daily dose, namely oestrogen plus MPA or oestrogen alone are put up in a calendar pack as is the normal practice with contraceptive tablets.

Optionally, tablets containing neither active ingredients (dummy tablets) can be provided for the days when neither ingredient is to be taken.

Accordingly the present invention provides a calendar pack containing pharmaceutical formulation doses for use in treating female scalp hair loss or in hormonal replacement therapy, comprising spaced locations corresponding to days of a menstrual cycle, dosage forms in a first series of said locations providing at each location of the series a daily dose of an oestrogen in pharmaceutically-administrable form without medroxyprogesterone acetate or its pharmacologically-active equivalent, and dosage forms at a second series of locations following the first series providing a daily dose of the oestrogen and a daily dose of medroxyprogesterone acetate or its pharmacologically-active equivalent in pharmaceutically-administrable form, wherein the total dosage of medroxyprogesterone acetate or its equivalent in the pack is at least 1mg and not more than 500mg per cycle, with the proviso that the said equivalent is not cyproterone acetate.

An example of such pack would be a bubble pack of the conventional kind having a tablet or other dosage form contained in a bubble at each of a series of space locations, normally positioned in a closed loop running round the edge of the pack.

The locations may be numered, normally 1 to 28.

The first series of bubbles may contain only one tablet or other dosage form per location containing oestrogen but not MPA. The first series of bubbles containing oestrogen only will preferably correspond to up to the first 21 days, e.g. the first 16 days of a 28 day cycle.

The second series of locations may then contain either one tablet or other dosage form containing both active ingredients or a pair of tablets or other dosage forms per location, one containing oestrogen and the other MPA. Where two dosage forms are provided they may of course be put in separate bubbles at the same day's location. The second series may comprise enough locations to correspond to up to the first 27 days of a cycle, preferably from the 16th to the 21st day.

If further locations are provided, they may be empty so that they can simply be burst in turn to mark the day's passing or may contain a dosage form containing neither active ingredient.

An example of a tablet formulation according to the present invention would, for instance, be:

| 40ug ethinyloestradiol | 70.0mg lactose |
| 10mg medroxyprogesterone acetate | 94.0mg corn starch |
| 10mg talcum | 3.2mg gelatin |
| | 2.8mg magnesium stearate |

It has been found that treatment described as above results in decreased hair fall, decrease in the greasiness of the hair, increased hair density together with the alleviation of the menopausal symptoms experienced by many women e.g. hot flushes, vaginal dryness, mild acne and greasy skin, depression, loss of confidence, decreased mental alertness and a general feeling of being unwell.

It has been found that patients treated with Medroxyprogesterone Acetate or its pharmacologically active equivalent, derivative or analogue and at least one oestrogen administered systemically as described in the Invention may unexpectedly benefit further from the addition of topical preparations applied directly to the scalp.

Therefore, it is proposed that the addition of topical preparations may enhance the effects of systemic preparations leading to the possible reduction in the dose of either the topical or systemic preparations to produce a synergistic therapeutic effect. For this reason it may be possible for the lower limits of the doses described previously to be reduced while maintaining the effectiveness of the preparations (systemic and topical) in the arrest and reversal of the common baldness process. Such a combination of therapy would be of considerable practical clinical value in allowing the minimum effective dose of treatment to be used.

CLINICAL EXAMPLES

Four patients aged 42-50 years had experienced hair loss beginning at the ages of 36-43 years for the first time. All patients had a regular menstrual cycle apart from one patient aged 45 years whose menopause had begun at the age of 37 years. Previous treatments, including iron tablets, yeast, various vitamins and standard hormone preparations (described below), had failed to arrest the hair fall which was progressive, although other menopausal symptoms had improved. The patients had received combinations of the following:

Premarin (Ayerst): containing conjugated

oestrogens (equine) 0.625mg or 1.25mg taken for 21 days starting on the 5th day of menstruation and then 7 tablet-free days.

Prempak (Ayerst): containing conjugated oestrogens (equine) 0.625mg or 1.25mg with norgestrel 0.5mg 7 tablets starting on the 5th day of menstruation and then 7 tablet-free days.

Premarin (Ayerst): containing conjugated oestrogens (equine) 0.625mg plus Duphaston (Duphar) dydrogesterone 10mg daily for 7 days from day 14.

Ovranette (Wyeth) containing ethinyloestradiol 30μg daily for 21 days combined with levonorgestrel 150μg daily for 21 days.

Brevinor (Syntex) containing ethinyloestradiol 35μg daily for 21 days combined with norethisterone 0.5mg daily for 21 days.

Ovysmen (Ortho-Cilag) containing ethinyloestradiol 35μg daily for 21 days with norethisterone 0.5mg for 21 days.

Norgeston (Schering) containing levonorgestrel 30μg continuously.

Following endocrine and trichological assessment, the patients were treated with:

Ethinyl-oestradiol:30μg - 40μg daily for 21 days of each cycle. MPA:5mg or 10mg daily from days 16 to 21 of the menstrual cycle.

Following the withdrawal bleed after 5-7 days, treatment was restarted.

Within 3 months each patient reported an improvement in hair condition, reduced greasiness and reduction in hair fall and an improvement in general wellbeing. Each patient also reported subsequently increased new hair growth and in particular the full growth previously of fine short hairs at the frontal margin.

The hair line had stopped receding and had moved forward with the growth of new hair. Treatment continued over 2-4 years with regular withdrawal bleeds, no pregnancies and with the retention of the hair growth achieved.

In the patients treated there were no unwanted side effects with normal hawmatology, liver function tests, renal function tests, general biochemistry and cervical smears repeated each 1-2 years remaining normal.

As will be appreciated, MPA can be replaced in the formulations of the present invention by any pharmacologically-active thereof at a corresponding dosage level. By this is meant any anti-androgen which has a progesterone-like action similar to MPA locally on scalp hair follicles, whilst at the same time does not significantly reduce SHBG levels. Suitable equivalents are allyloestrenol and gestronol. Hydroxprogesterone and dydrogesterone may be effective in some patients, but the latter only if the local dosage is high enough and certainly higher than 10 x 7 i.e. 70mg per month described above which was administered systemically.

Cyproterone acetate (CPA) is an equivalent of MPA, but since formulations similar to those of the present invention are described and claimed separately in my co-pending application No. (Agents' Ref:                )CPA formulations are specifically excluded from the scope of this application.

Additional information regarding an unexpected beneficial effect upon systemically administered Medroxyprogesterone Acetate or its pharmacologically active equivalent and at least one oestrogen by the application of topical preparations.

The following topical preparations may be used in women in combination with the Invention as previously described in a volume of 1-10ml in divided doses to the affected sites on the scalp:

1) Preparation 1:

One or more of:

Oestradiol benzoate 0.2%          (range 0.001-5%)

Medroxyprogesterone Acetate 0.2%     (range 0.001-5%)

3,3-5 Triiodo-L-Thyronine Free Acid 20ug/3ml of solution up to 1%.

Or metabolites or derivatives or analogues thereof.

2) Preparation 1 plus vasodilator:

One or more of:

Oestradiol Benzoate 0.1%       . (range 0.001-5%)

Medroxyprogesterone Acetate 0.1%     (range 0.001-5%)

3,3-5 Triiodo-L-Thyronine Free Acid 20ug/3ml of solution up to 1%.

Plus a vasodilator e.g:

a) Phentolamine Mesylate (as Rogitine, CIBA)
0.1%                                 (range 0.001-10%)
b) Isoprenaline Hydrochloride 0.04%    (range 0.001-10%)
c) Minoxidil 0.1%-4%                 (range 0.001-10%)

Or metabolites or derivatives or analogues thereof.

The addition of one or more vasodilators applied topically or given systemically could be included in the Invention. The nature of the vasodilators is not critical.

3) <u>Preparation 2 plus "2nd Messenger"</u>

One or more of:

Oestradiol Benzoate 0.2%        (range 0.001-5%)

Medroxyprogesterone Acetate 0.2%        (range 0.001-5%)

3,5 Triiodo-L-Thyronine Free Acid 20ug/3ml of solution up to 1%

Vasodilator as described in 2, a,b and/or c above

"2nd Messenger" as:

One or more of:

Cyclic AMP Free Acid 0.1%        (range 0.00000000001-20%)

Cyclic AMP Sodium 0.05%        (range 0.00000000001-20%)

N6, 2-0-Dibutyryl Adenosine 3,5,Cyclic Monophosphate  (a long acting synthetic form of Cyclic AMP) 0.01%    (range 0.00000000001-20%)

ATP Magnesium 0.1%        (range 0.00000000001-20%)

Choline Theophyllinate (phosphodiesterase inhibitor) 0.2%
                       (range 0.0001-20%)

Caffeine (phosphodiesterase inhibitor)0.2% (range 0.0001-20%)

Other "2nd Messengers" may be included according to the Invention e.g.

One or more of:

Cyclic GMP Free Acid 0.01%        (range 0.00000000001-20%)

Cyclic GMP Sodium 0.1%        (range 0.00000000001-20%)

N2,2-0-Dibutyryl Guanosine 3,5, Cyclic Monophosphate (a long acting synthetic form of cyclic GMP) 0.01%    (range 0.00000000001-20%)

5,GDP

5,GTP

5,G Tetra P

5,Guanylyl Imidodiphosphate (a long acting form of GTP)

Inosine 3,5, Cyclic Monophosphate, Diphosphate, Triphosphate

Thymidine 3,5, Cyclic Monophosphate, Diphosphate, Triphosphate

Uridine 3,5, Monophosphate, Diphosphate, Triphosphate

Or other "2nd Messenger" systems, derivatives or analogues thereof at a dose range of 0.0000000000.1-20% or phosphodiesterase inhibitors.

4) Preparation 1,2, or 3 plus enzymes of the Embden Meyerhoff Parnass Pathway, the Pentose Phosphate Shunt or the Tricarboxylic Acid cycle to include:

One or more of:

| | |
|---|---|
| Phosphorylase 625 IU/L | (range 1-100,000) |
| Hexokinase 1,000 IU/L | (range 1-100,000) |
| Glucose-6-Phosphate Dehydrogenase 1,000 IU/L | (range 1-100,000) |
| Phosphofructokinase 1,000 IU/L | (range 1-100,000) |

Or other enzyme systems at a dose range of 1-100,000 IU/L.

5) Preparations 1,2,3 or 4 plus:

Para Amino Benzoic Acid or salts or derivatives thereof 0.1% to 0.3%
(range 0.0001-20%)

6) Preparations 1,2,3,4 or 5 plus:

One or more other anti-androgens e.g.

| | |
|---|---|
| Spironolactone 0.1-3% | (range 0.001-20%) |
| Deoxycorticosterone 0.2% | (range 0.001-20%) |
| Cimetidine 0.1% | (range 0.001-20%) |
| Desogestrel 0.1% | (range 0.001-20%) |
| Megestrol Acetate 0.1% | (range 0.001-20%) |
| Ethynodiol Diacetate 0.1% | (range 0.001-20%) |
| Cyproterone Acetate 0.1% | (range 0.001-20%) |

Lynoestrenol 0.1%                    (range 0.001-20%)

Norethisterone 0.1%                  (range 0.001-20%)

Levonorgestrel 0.1%                  (range 0.001-20%)

Flutamide 0.1%                       (range 0.001-20%)

Progesterone 0.1%                    (range 0.0001-20%)

Azelaic Acid 0.1%                    (range 0.001-20%)

Testolactone 0.1%                    (range 0.001-20%)

Danazol 0.1%                         (range 0.001-20%)

Or other anti-androgens or metabolites or derivatives or analogues thereof.

7)  Preparations 1,2,3,4,5 or 6 plus:

One or more of:

Immunosupressives Amino Acids such as those normally found in the hair Glucose or other energy source.


When the topical preparations as set out above (some components of which may be given systemically e.g. Triiodothyronine as the sodium salt given orally in the range of 1ug-300ug daily, more preferable 20ug three times daily) are administered in addition to the systemic cyclical anti-androgen therapy as described in the Invention there has resulted a clear increase in the rate of growth of scalp hair. Typically patients have reported that prior to the addition of the topical preparations they would normally cut their hair each 2-3 months to maintain the same style. However, the addition of the above preparations has resulted in hair being required to be cut each 4-6 weeks to maintain the same hair style. The rate of growth is increased particularly in the temporal and occipital areas with an increase in rate of growth in these areas being noted before that in the frontal and vertex regions. Typically the rate of hair growth has increased by approximately 25-200% during the first 3-6 months of treatment.

CLAIMS:

1.    A pharmaceutical formulation for use in treating female scalp hair loss or in hormonal replacement therapy which comprises medroxyprogesterone acetate or its pharmacologically-active equivalent and at least one oestrogen, wherein when prepared in unit dosage form the formulation is adapted to administer at least 1mg and not more than 500mg of metroxyprogesterone acetate or its pharmacologically-active equivalent per month, with the proviso that the said equivalent is not cyproterone acetate.

·2.    A formulation as claimed in claim 1 further comprising a pharmaceutically-acceptable carrier, diluent or excipient.

3.    A formulation as claimed in claim 1 or 2 in the form of a unit dosage form for oral administration

4.    A formulation as claimed in claim 3 which provides from 1 to 50mg of medroxyprogesterone acetate per unit dosage form.

5.    A formulation as claimed in any preceding claim wherein the or each oestrogen present is ethinyloestradiol or its pharmacologically-active equivalent.

6.    A formulation as claimed in claim 5 when appendant to either claim 3 or claim 4 wherein the formulation provides from 10 to 60ug per unit dosage form of ethinyloestradiol.

7.    A calendar pack containing pharmaceutical formulation doses for use in treating female scalp hair loss or in hormonal replacement therapy, comprising spaced locations corresponding to days of a menstrual cycle, dosage forms in a first series of said locations providing at each location of the series a daily dose of an oestrogen in pharmaceutically-administrable form without medroxyprogesterone acetate or its pharmacologically-active equivalent, and dosage forms at a second series of locations following the first series providing a daily dose of the oestrogen and a daily dose of medroxyprogesterone acetate or its pharmacologically-active equivalent in pharmaceutically-administrable form, wherein the total dosage of medroxyprogesterone acetate or its equivalent in the pack is at least 1mg and not more than 500mg per cycle, with the proviso that the said equivalent is not cyproterone acetate.

8.    A pack as claimed in claim 7, wherein the oestrogen in ethinyloestradiol or its pharmacologically-active equivalent.

9.    In combination, a pharmaceutical formulation as claimed in any one of claims 1 to 8, preferably together with a topical treatment for the stimulation of hair growth a container therefor, and instructions for the use of the pharmaceutical formulation in the treatment of female scalp hair loss or in hormonal replacement therapy.

10. The use of a combination of a pharmaceutical formulation as claimed in any one of claims 1 to 6 preferably together with a topical treatment for the stimulation of hair growth for promoting female scalp hair growth.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | UNLISTED DRUGS, vol. 23, no. 2, February 1971, page 27, point m, Chatham, N.J., US; <br> * Page 27, point m, "Verafem" * | 1-10 | A 61 K   31/57 <br> A 61 K    7/06 |
| X | UNLISTED DRUGS, vol. 15, no. 11, November 1963, page 87, point h, Chatham, N.J., US; <br> * Page 87, point h, "Nogest" * | 1-10 | |
| X | ROTE LISTE, 1969, pages 1377 and 1391, Editio Cantor, Aulendorf/Württ., DE; <br> *   Page  1377,  "Weradys";  page 1391, "Zyklo-Farlutal" * | 1-10 | |
| X | EP-A-0 033 164  (IRMGARD VON KISTOWSKI) <br> * Page 5, claims 1-5 * | 1-10 | TECHNICAL FIELDS SEARCHED (Int. Cl.4) |
| A | US-A-3 942 641  (EUGENE J. SEGRE) <br> *  Column 9, line 38 - column 10, line 38; claims 1-7 * | 1-10 | A 61 K |
| A | DE-A-2 757 024  (JÜRGEN TAMM) | 1-10 | |

The present search report has been drawn up for all claims

| Place of search | Date of completion of the search | Examiner |
|---|---|---|
| THE HAGUE | 22-08-1985 | BRINKMANN C. |